# EUROPEAN PATENT SPECIFICATION

(11) **EP 3 019 077 B1**
(45) Date of publication and mention of the grant of the patent: **21.10.2020**
(21) Application number: 14823693.8
(22) Date of filing: 27.06.2014
(51) Int. Cl.: A61B 5/02, A61B 7/04, A61B 5/024

(54) **CLASSIFYING HEART SOUNDS**
KLASSIFIZIERUNG VON HERZTÖNEN
CLASSIFICATION DES BRUITS DU COEUR

(30) Priority: 11.07.2013 SE 1350864
(43) Date of publication of application: 18.05.2016
(73) Proprietor: Coala-Life AB, 114 51 Stockholm (SE)
(72) Inventor: GHARAHBAGHI, Arash, 587 35 Linköping (SE); ASK, Per, 585 91 Linköping (SE)
(74) Representative: Kransell & Wennborg KB
(86) International application number: PCT/SE2014/050804
(87) International publication number: WO 2015/005850

(56) References cited:
- WO-A1-2008/000254
- WO-A1-2009/027804
- WO-A1-2009/098312
- WO-A1-2009/138932
- WO-A1-2012/025815
- CN-A- 103 165 127
- KR-A- 20110 127 785
- US-A1- 2003 055 352
- US-A1- 2011 282 654
- ZHONGWEI JIANG ET AL.: 'A New Approach on Heart Murmurs Classification with SVM Technique' INTERNATIONAL SYMPOSIUM ON INFORMATION TECHNOLOGY CONVERGENCE 2007, pages 240 - 244, XP055308452
- CHOI S ET AL.: 'Cardiac sound murmurs classification with autoregressive spectral analysis and multi-support vector machine technique' COMPUTERS IN BIOLOGY AND MEDICINE vol. 40, no. 1, 2010, pages 8 - 20, XP026857531
- GHAREHBAGHI A ET AL.: 'Detection of systolic ejection click using time growing neural network' MEDICAL ENGINEERING & PHYSICS vol. 36, no. 4, April 2014, pages 477 - 483, XP028836063

## Description

### TECHNICAL FIELD

The invention relates in general to the field of analysing phonocardiograms, and in particular, to a device and computer program for classifying heart sounds.

### BACKGROUND

A phonocardiogram is a recording of heart sounds using an acoustical sensor. One heart beat is a cardiac cycle. The cardiac cycles repeat itself throughout life. The pace of the cardiac cycles can vary depending on the respiration and level of strain on the body. Each cardiac cycle can be divided into two parts, the systolic duration and the diastolic duration.

Valvular heart diseases often affect the heart sounds by adding extra sounds which are called abnormal heart sounds. Heart murmurs are the most prevalent abnormal heart sounds. This murmur could be created by chronically or acutely diseased hearts. One disease which can cause heart murmurs is aortic stenosis.

Each heart disease has a unique temporal template along with specific frequency contents distributed over time of the phonocardiogram of said heart. By extracting the spectral energies over time in cardiac cycles, conclusions regarding what heart disease a heart has and even the degree of said heart disease could be determined. Various single resolution or multi resolution time-frequency representation of the signal i.e. wavelet analysis could be employed to extract the spectral energies over time in a cardiac cycle.

Analysing heart sounds often comprises a classification of the heart sound in several steps. In a first step, the heart sound is classified as normal or abnormal. Abnormal heart sounds could for example comprise murmur. There are two types of murmur, innocent murmur and pathological murmur. A second classification step involves classifying the murmur as innocent or pathological.

Pathological murmur often occurs in a systolic part of a cardiac cycle. The pathological murmur could differentiate between cardiac cycles from the same phonocardiogram. The murmur could for example show different amplitude or duration in different cardiac cycles.

One problem with conventional analysing devices performing methods for analysing heart sounds in order to detect abnormal heart sounds comprising pathological murmur, such as the wavelet analysis method, is that the performed methods are suitable for detecting murmur where said murmur signals are stationary in each cardiac cycle.

Since pathological murmur often has a varying amplitude and a varying duration in each cardiac cycle, conventional analysis could accidently classify an abnormal heart sound comprising non-pathological murmur as a pathological heart sound. This causes many patients to unnecessarily visit specialists. The large number of patients remitted to specialists puts a strain on the valuable available time of the specialists. In addition, conventional analysing methods could accidently classify an abnormal heart sound comprising pathological murmur as innocent heart sound which could put the patients in question in danger.

There is a desire to improve an analysing device performing methods for analysing phonocardiograms. It is an object of the disclosure to provide a device performing an improved method for analysing heart so that it will be possible to identify and remit patients with pathological murmur to specialists, while patients with innocent murmur will not be remitted to specialists.

US 2003/0055352 A1 discloses a method of diagnosing pathologic heart conditions in which a time series of heart sounds is filtered and parsed into a sequence of individual heart cycles. A systolic interval as well as systolic sub-intervals are identified for each heart cycle. An energy value is computed for the systolic subinterval of one or more heart cycles. The energy value computed is proportional to the energy level associated with the filtered series of heart sounds. A composite energy value is then computed for the systolic sub-intervals of one or more heart cycles and compared to a threshold level in order to distinguish between a normal heart and a pathologic heart.

### SUMMARY

It is an object of the present invention to eliminate or at least mitigate at least some of the above mentioned problems relating to devices arranged for analysing phonocardiograms.

This is achieved by a device for classifying heart sounds presented in a phonocardiogram according to claim 1. The device comprises a memory unit and a processing unit. The processing unit is arranged for classifying heart sounds presented in a phonocardiogram by performing a series of steps. In a first step a phonocardiogram comprising at least one cardiac cycle is received. A first point in relation to a first heart sound in said cardiac cycle, presented in the phonocardiogram, is determined. A second point in relation to a second heart sound in said cardiac cycle, presented in the phonocardiogram, is determined. A first analysis on said heart sound is performed over a first time window, said analysis comprising the step of extracting spectral energies from said phonocardiogram and said first time window having a starting point essentially the same as said first point or an ending point essentially the same as said second point and having a duration of a first predetermined time, wherein said first predetermined time is shorter than a time period between said first point and said second point. When at least one further time window has a duration of a further predetermined time, wherein said further predetermined time is shorter than said time period between said first point and said second point, at least one further analysis is performed on said heart sound over at least one further time window, said analysis comprising the step of extracting spectral energies from said phonocardiogram, said at least one further time window having essentially the same starting point as said first point or essentially the same ending point as said second point and having a duration of a further predetermined time. Said further predetermined time of said further time window is the sum of the duration of said previous time window and a predetermined time period. Further, said heart sound, presented in the phonocardiogram, is classified as a non-pathological or a pathological heart condition on the basis of said first and at least one further analysis of the heart sound over said first and at least one further time window.

The device arranged to perform the disclosed method is suitable for extracting spectral energies from certain discriminative frequency bands, said extracted spectral energies provide an improved basis for discrimination between pathological and innocent murmur. The device performing the analysis method is especially suitable for extracting spectral energies which provide an improved basis for discrimination between pathological and innocent murmur, in particular when the murmur is non-stationary, that is, wherein the murmur varies in amplitude and duration between different cardiac cycles in a phonocardiogram.

According to an aspect of the disclosure, said first and second points can be chosen based on what pathological condition that should be investigated. If one particular pathological condition is known to cause murmur at the beginning of the systolic duration, but after the first sound of the systolic duration, said first point can be chosen to be placed where said first sound terminates in said systolic duration. In the same manner, said second point can be chosen at a selected point in a cardiac cycle, where said selected position depends on which particular pathological condition that should be investigated.

According to another aspect of the disclosure, said first predetermined time and predetermined time period are chosen by means of a database comprising phonocardiograms from several hearts wherein said phonocardiograms have been confirmed to belong in one out of three groups; no murmur, innocent murmur and pathological murmur. Based on this data, an optimal first predetermined time and an optimal predetermined time period can be determined in order to obtain an optimal analysis of said phonocardiogram. In addition, discriminative frequency bands over which said spectral energies are extracted are determined by means of said database comprising phonocardiograms from several hearts, said database forms a training database.

In the device, performing the above disclosed method, the so called Growing Time Window analysis, the contents of said first time window will be taken into consideration for each further time window in the analysis. Said first time window can be placed anywhere on the signal of the cardiac cycle, for example in the beginning of the systolic duration, at the end of the systolic duration or somewhere in the middle of the systolic or diastolic duration.

The above described device performs the method time efficiently, requires a low calculation capacity and provides an accurate analysis of the heart sound. The above described device is arranged for performing a simplified analysis of heart sounds. In addition an accurate classification of heart sounds can be received without a specialist examining the patient. The device can receive the phonocardiograms from relatively simple apparatuses used to record phonocardiograms.

The object of the disclosure is also achieved by a computer program comprising computer program code which when executed causes a device to perform classification of hear sound presented in a phonocardiogram as disclosed above. The computer program may be executed in a processor at a site where a patient is examined. Said patient could then accurately be examined at a site even when no specialist is present.

According to one aspect, said computer program comprising computer program code can be provided in a portable device, such as a mobile phone.

According to one aspect, said first and said at least one further analysis of said heart sound over said first and said at least one further time windows comprise the step of extracting spectral energies from said heart sound.

According to one aspect, said classification of the heart sound into a first or second heart condition involves using a support vector machine method.

The Growing Time Window analysis in combination with said Support Vector Machine (GTSVM) forms an accurate analysis method for determining whether a heart sound comprises pathological murmurs or not. The extracted spectral energies which are extracted by the Growing Time Window analysis forms a good basis for applying said Support Vector Machine in order to be able to classify said murmur into a pathological murmur or a non-pathological murmur.

According to one aspect, said phonocardiogram comprises several cardiac cycles. According to this embodiment said method comprises the following steps: deriving a result from said analysis of the heart sound from said first and at least one further time windows for each cardiac cycle, deriving an average result on the basis of the result from each of the analysis of the cardiac cycles and classifying the heart sound into a first or second heart condition on the basis of said derived average result from said analysis of the heart sound from the at least two time windows. By analysing multiple cardiac cycles and using an average result based on the analysis of each cycle, a more accurate result can be achieved.

According to one aspect, said phonocardiogram comprises several cardiac cycles wherein the classification of the heart sound into a first or second heart condition for each cardiac cycle is used in order to determine an overall classification for the phonocardiogram. By classifying each cardiac cycle separately and thereafter use the result from the classification from each cardiac cycle, an alternative method for using the result from all the cardiac cycles is achieved. By using the result from several cardiac cycles, a more accurate result is achieved.

According to one aspect, the processing unit of said device is additionally arranged to perform the steps of: assuming that said pathological condition is a predetermined pathological condition, receiving said phonocardiogram comprising at least one cardiac cycle, determining a third point in relation to a first heart sound in said cardiac cycle, determining a fourth point in relation to a second heart sound in said cardiac cycle, performing a first analysis on said heart sound over a time window, said time window having a starting point essentially the same as said third point or having an ending point essentially the same as said fourth point and having a duration of a second predetermined time. When one at least further time window, having a duration of said second predetermined time, is within a time interval created between said third and fourth points, performing at least one further analysis on said heart sound over said at least one further time window, said at least one further time window having a starting point a predetermined time period later than said starting point of said previous time window or having an ending point a predetermined time period earlier than said ending point of said previous time window. Further, said method comprises the step of confirming or dismissing said predetermined pathological condition of said heart sound on the basis of said first and at least one further analysis of the heart sound over said first and at least one further time window.

Said second predetermined time and said predetermined time period are chosen by means of a database comprising phonocardiograms from several hearts wherein said phonocardiograms have been confirmed to belong in one out of three groups; no murmur, innocent murmur and pathological murmur. Based on this training data, an optimal second predetermined time and an optimal predetermined time period can be determined in order to obtain an optimal analysis of said phonocardiogram.

According to one aspect, said processing unit of the device is additionally configured to perform the steps of: receiving said phonocardiogram comprising at least one cardiac cycle, determining a third point in relation to a first heart sound in said cardiac cycle, determining a fourth point in relation to a second heart sound in said cardiac cycle, performing a first analysis on said heart sound over a time window, said time window having a starting point essentially the same as said third point or having an ending point essentially the same as said fourth point and having a duration of a second predetermined time. When at least one further time window, which has a duration of said second predetermined time, is within a time interval created between said third and fourth points, performing at least one further analysis on said heart sound over said at least one further time window, said at least one further time window having a starting point a predetermined time period later than said starting point of said previous time window or having an ending point a predetermined time period earlier than said ending point of said previous time window, grading the severity of said predetermined pathological condition of said heart sound on the basis of said first and at least one further analysis of the heart sound over said first and at least one further time window.

Said second predetermined time and said predetermined time period are chosen by means of a database comprising phonocardiograms from several hearts wherein said phonocardiograms have been confirmed to belong in one out of three groups; no murmur, innocent murmur and pathological murmur. The phonocardiograms being confirmed to belong to said predetermined pathological murmur group has each been further divided into confirmed pathological conditions. Each phonocardiogram with a specific predetermined pathological condition has further been graded based on the severity of said predetermined pathological condition. Based on this training data, an optimal second predetermined time and an optimal predetermined time period can be determined in order to obtain an optimal analysis of said phonocardiogram.

According to one aspect of the disclosure, said first and at least one further analysis of said heart sound over said first and at least one further time windows tw1, twn comprises the step of extracting spectral energies from said heart sound.

According to one aspect of the disclosure, said confirmation or dismissal of said predetermined pathological condition of said heart sound involves using a support vector machine method.

Said support vector machine method is adjusted with the help of said database comprising phonocardiograms from several hearts, a training database, wherein each phonocardiogram has been classified into predetermined pathological conditions, in order to achieve the best possible result of said method.

According to one aspect of the disclosure, said grading of the severity of said predetermined pathological condition of said heart sound involves using a wavelet analysis or stepwise regression analysis.

Said wavelet analysis is adjusted with the help of said training database comprising phonocardiograms from several hearts, wherein each phonocardiogram has been classified into predetermined pathological conditions, and wherein each phonocardiogram which has been determined to show a predetermined pathological condition has been graded in terms of severity, in order to achieve the best possible result of said analysis.

Said stepwise regression analysis is adjusted with the help of said database comprising phonocardiograms from several hearts, wherein each phonocardiogram has been classified into predetermined pathological conditions, and wherein each phonocardiogram which has been determined to show a predetermined pathological condition has been graded in terms of severity, in order to achieve the best possible result of said analysis.

According to one aspect of the disclosure, said pathological condition is aortic stenosis.

According to one aspect of the disclosure, said pathological condition is aortic regurgitation.

According to one aspect of the disclosure, said pathological condition is mitral insufficiency.

According to one aspect of the disclosure, said first or third points and said second or fourth points are within the systolic duration of a cardiac cycle.

According to one aspect of the disclosure, said first or third points and said second or fourth points are within the diastolic duration of a cardiac cycle.

Each pathological condition which causes murmur, causes murmur either in the systolic duration or in the diastolic duration of said cardiac cycle. Depending on where in the cardiac cycle said pathological condition causes murmur to occur, a suitable part of said cardiac cycle is analyzed.

According to one aspect of the disclosure, said device is arranged to perform analysis of heart sounds on at least two sequentially occurring cardiac cycles of said phonocardiogram. In order to receive an accurate result from said analysis each cardiac cycle in a phonocardiogram can be analyzed.

According to one aspect of the disclosure, said analysis of heart sounds is performed on at least two intermittently occurring cardiac cycles of said phonocardiogram. In order to receive an accurate result from said analysis, but with less strain on said processor, a selection of a total number of cardiac cycles in said phonocardiogram can be analyzed.

### BRIEF DESCRIPTION OF THE DRAWINGS

The above mentioned features and advantages of the present invention will become readily apparent to those skilled in the art by the following detailed description of exemplary embodiments thereof with reference to the accompanying drawings wherein:
Fig. 1 schematically illustrates an example of a device and a system according to the invention,
Fig. 2a schematically illustrates a cardiac cycle of a normal heart sound,
Fig. 2b schematically illustrates a cardiac cycle of an abnormal heart sound,
Fig. 2c schematically illustrates a number of time windows distributed over a cardiac cycle according to one aspect of the invention,
Fig. 2d schematically illustrates the number of time windows distributed over a cardiac cycle illustrated in Fig. 2c in greater detail,
Fig. 2e schematically illustrates a number of time windows distributed over a cardiac cycle according to one aspect of the invention,
Fig. 2f schematically illustrates a number of time windows distributed over a cardiac cycle according to one aspect of the invention,
Fig. 2g schematically illustrates a number of time windows distributed over a cardiac cycle according to one aspect of the invention,
Fig. 3a schematically illustrates a method according to the invention,
Fig. 3b schematically illustrates a method according to the invention.
Fig. 4 schematically illustrates block diagram of a device for classifying heart sounds.

### DETAILED DESCRIPTION

The figures are schematic and simplified for clarity reasons to show features which are essential to the understanding of the invention, while leaving out less significant details. Throughout the following description, the same reference numerals are used for identical or corresponding parts or steps.

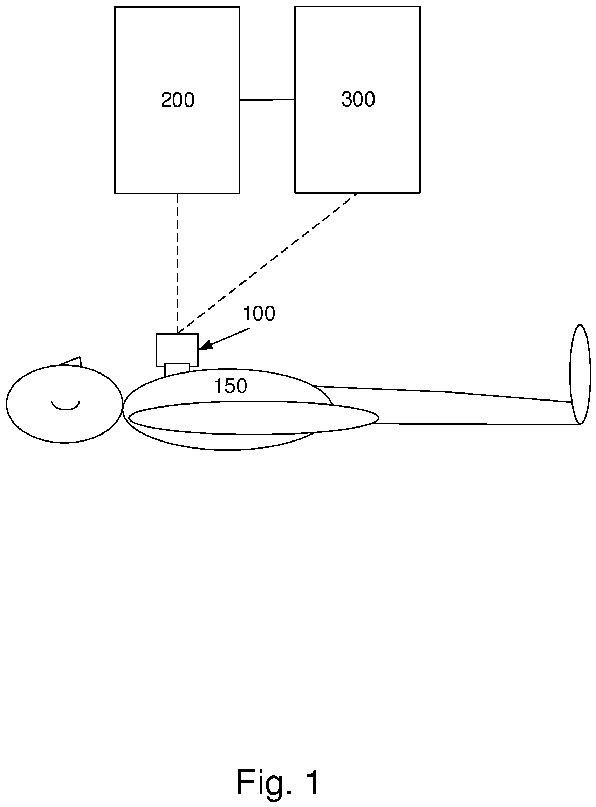

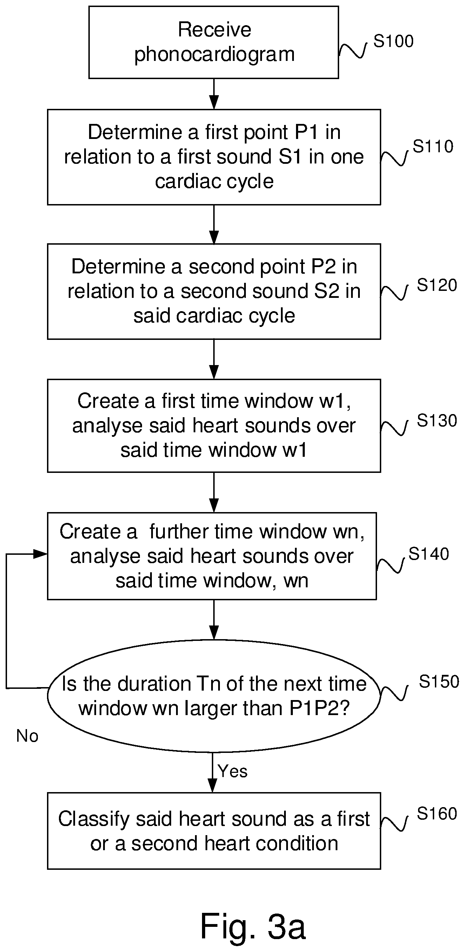

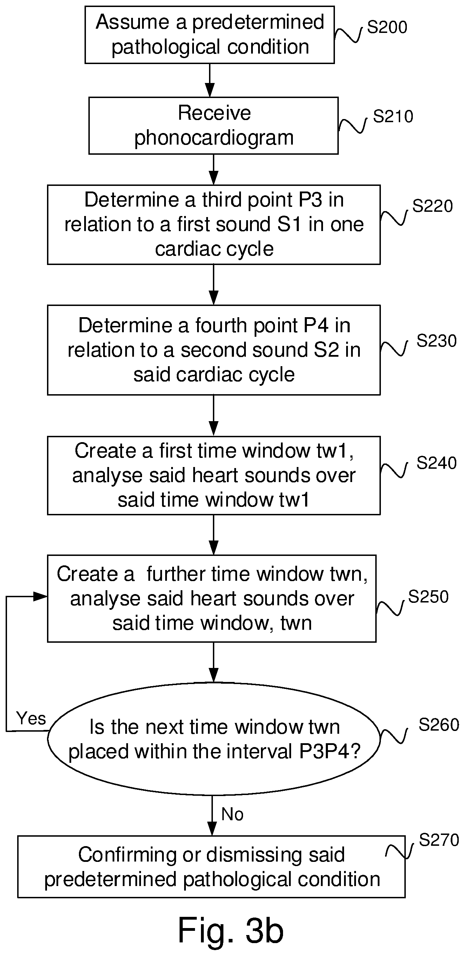

Fig. 1 shows a device according to one embodiment of the invention. A sensor 100 is placed on a patient 150 in a position suitable for detecting a phonocardiogram of the patient 150. The sensor 100 comprises memory unit for storing the phonocardiogram or means for real time transmission of said phonocardiogram to an external memory unit. The sensor comprises a transmitter. Said detected phonocardiogram is according to one embodiment transmitted to a processor 200 wireless or alternatively via a link. Said processor 200 perform the analysis of the phonocardiogram by applying the method of Figures 3a or 3b which will be described in further detail below.

Said processor 200 is according to one embodiment connected to a communication device 300. Said communication device 300 is according to one embodiment arranged to display the results from the analysis method performed in said processor 200. According to one embodiment, said communication device 300 is a display. According to one embodiment, said communication device 300 comprises control means, such as a key pad, via which a user can control said processor 200 via said communication device 300. Said communication device 300 can according to one embodiment receive a phonocardiogram wirelessly or via a link and transmit said phonocardiogram to said processor 200.

Said processor 200 and said communication device 300 could be connected wirelessly or via a link. According to one embodiment, said processor 200 and communication device 300 form parts of one unit (not shown). According to one embodiment, said processor 200 and said communication device 300 form parts of two different units.

According to one embodiment, said sensor 100 and said processor form one unit. Said sensor is, according to one embodiment a mobile phone.

In accordance with this inventive analysis method for classifying heart sounds, it is essential to establish where in a cardiac cycle the heart sound to be analysed occurs. It is also essential to establish that the frequency contents of the pathological murmur differ from the frequency contents of the innocent murmur.

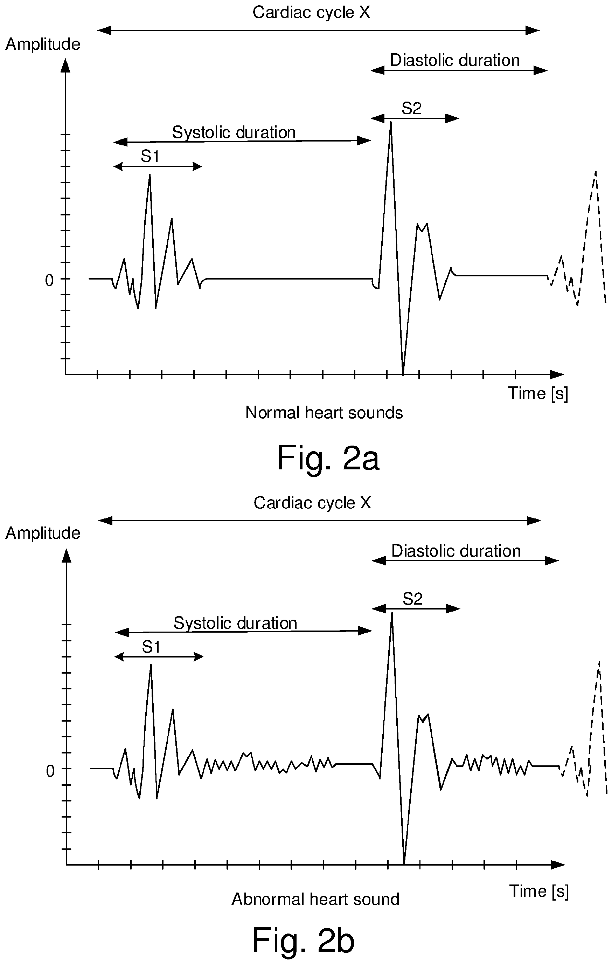

Fig. 2a discloses a cardiac cycle X comprising normal heart sounds. A cardiac cycle X can be divided into two durations, the systolic duration and the diastolic duration. These durations are defined by the construction of the heart and its valves. When the valves in the heart close, a sound is created. Each cardiac cycle X comprises two main sounds, a first sound S1 when two of the four valves in the heart closes at the beginning of the systolic duration and a second sound S2 when the other two valves in the heart closes, at the start of the diastolic duration. S1 could typically last for 0.1 second. For a phonocardiogram of a normal heart, there is no sound after S1 until said second sound S2 is heard at the beginning of the diastolic duration. S2 could typically last for 0.1 second. The period of silence in between S1 and S2 and S2 and S1 could typically last for around 0.25 seconds.

Fig. 2b discloses a cardiac cycle X comprising abnormal heart sounds. In this example, extra heart sounds are sound in between the first sound S1 and the second sound S2, and also in between the second sound S2 and the first sound S1. This extra heart sound is called murmur. Said murmur could be caused by a diseased heart or by a normal heart. Said murmur could be classified as pathological or innocent murmur. Certain pathologies murmur occurs at a specific part of said cardiac cycle X. Murmur during the systolic part of the cardiac cycle X is by far the most important and prevalent abnormal heart sound. This murmur could be caused both by a diseased and by a normal heart.

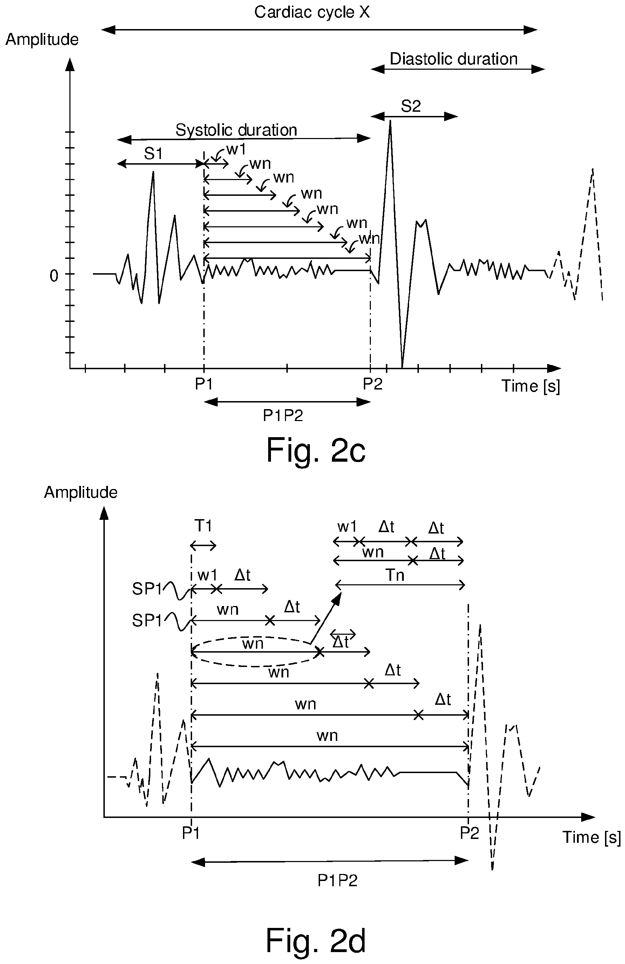

Fig. 2c discloses a diagram of a number of time windows distributed over a cardiac cycle X according to one aspect of the invention. The method according to claim 1 used to analyze murmur in a cardiac cycle X is described in relation to said diagram. A phonocardiogram comprising one cardiac cycle X comprising murmur is displayed. As a first step, a first point P1 in relation to a first heart sound S1 in said cardiac cycle X is determined. Thereafter, a second point P2 is determined, said second point P2 being determined in relation to a second heart sound S2 in said cardiac cycle X. A first time window w1 is created, said first time window w1 having a duration of a first predetermined time T1, said first predetermined time T1 being shorter than a time period P1P2 between said first point P1 and said second point P2. Said time window w1 has a starting point SP1 of essentially said first point P1. A first analysis is performed on said heart sounds over said first time window w1. Further, at least one further time window wn with a duration of a predetermined time Tn, wherein Tn is shorter than the time period P1P2 is created. Said at least one further time window wn has a starting point SP1 of essentially said first point P1. Said predetermined time Tn is the sum of the duration of said previous time window w1, wn and a predetermined time period Δt. At least one further analysis is performed on said heart sounds over said at least one further time window wn. Additional time windows wn are created and analysed, until said time window wn has a duration of a predetermined time Tn equal to, or exceeding, said time period P1P2.

Based on the results from the analysis above on said time windows w1, wn a classification is performed wherein said heart sounds are classified as a first or second heart condition.

Fig. 2d discloses what is disclosed in fig. 2c in a zoomed format. In figure 2d, the predetermined time Tn, which is the duration for a certain time window wn is shown.

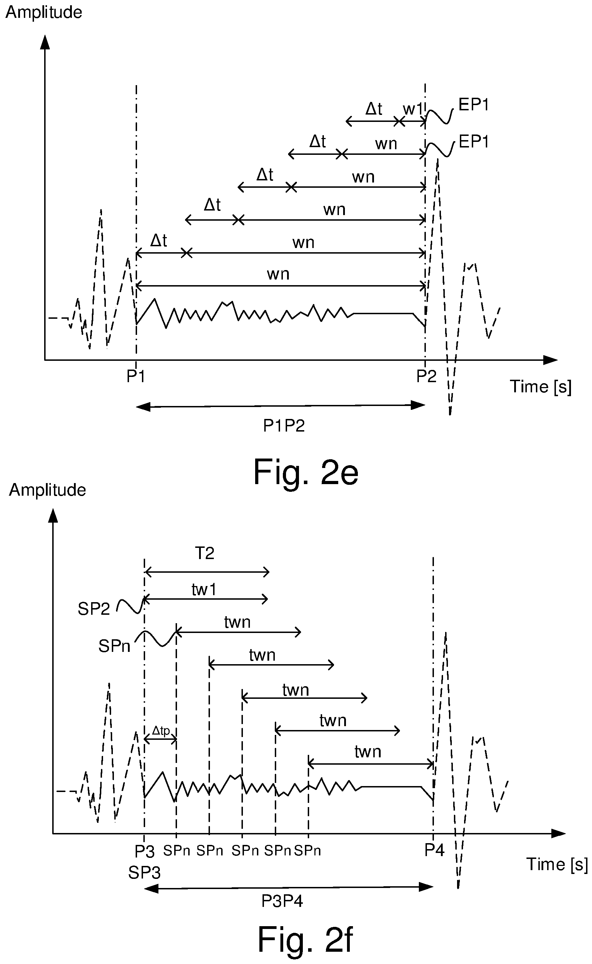

Fig. 2e discloses a diagram comprising a number of time windows distributed over a cardiac cycle X according to one aspect of the invention. The method according to claim 1 used to analyze murmur in a cardiac cycle X is described in relation to said diagram. A phonocardiogram comprising one cardiac cycle X is displayed. As a first step, a first point P1 in relation to a first heart sound S1 in said cardiac cycle X is determined. Thereafter, a second point P2 is determined in relation to a second heart sound S2 in said cardiac cycle X is determined. A first time window w1 is created, said first time window w1 having a duration of a first predetermined time T1, said first predetermined time T1 being shorter than a time period P1P2 between said first point P1 and said second point P2. Said time window w1 has an ending point EP1 essentially the same as said second point P2. A first analysis is performed on said heart sounds over said first time window w1. Further, at least one further time window wn with a duration of a predetermined time Tn, wherein Tn is shorter than the time period P1P2, is created. Said at least one further time window wn has an ending point EP1 essentially the same as said second point P2. The duration of said second time window is said previous time window w1, wn plus a predetermined time period Δt. An at least one further analysis is performed on said heart sounds over said second time window wn.

Additional time windows wn are created and analysed, until said time window wn has a duration Tn of, or exceeding, said time period P1P2.

The analysis method described above can be performed on each cardiac cycle X of a phonocardiogram.

Based on the results from the analysis above on said time windows w1, wn a classification is performed wherein said heart sounds are classified as a first or second heart condition.

Fig. 2f discloses a diagram comprising a number of time windows distributed over a cardiac cycle X according to one aspect of the invention.The method according to claim 8 or 9 used to analyze murmur in a cardiac cycle X is described in relation to said diagram. Said method is used to further analyze said confirmed pathological murmur in a cardiac cycle X.

If the detected murmur is classified as pathological murmur, a further analysis on the phonocardiogram is performed. This is in order to determine which pathological condition said heart from which said phonocardiogram is recorded shows. Said further analysis is schematically displayed in fig. 2f.

Said phonocardiogram comprises one cardiac cycle X where pathological murmur is displayed. A certain predetermined pathological condition is assumed. Further, a third point P3 in relation to a first heart sound S1 in said cardiac cycle X is determined. Thereafter, a fourth point P4 in relation to a second heart sound S2 in said cardiac cycle X is determined. A first analysis on said heart sound over a time window tw1 is performed, said time window tw1 having a starting point SP3 essentially the same as said third point P3 and having a duration of a second predetermined time T2. At least one further analysis on said heart sounds over at least one further time window twn is performed, said time window twn having a starting point SPn essentially a predetermined time period Δtp later than said starting point SP2 of said previous time window tw1, twn and having a duration of said second predetermined time T2.

Additional time windows twn are created and analyses on the heart sound over said additional time windows twn are performed, until said time window twn has an ending point EP2 essentially the same as or later than said fourth point P4.

Based on the results from the analysis above on said time windows tw1, twn a confirmation or a dismissing of said assumed predetermined pathological condition of said heart sound is made.

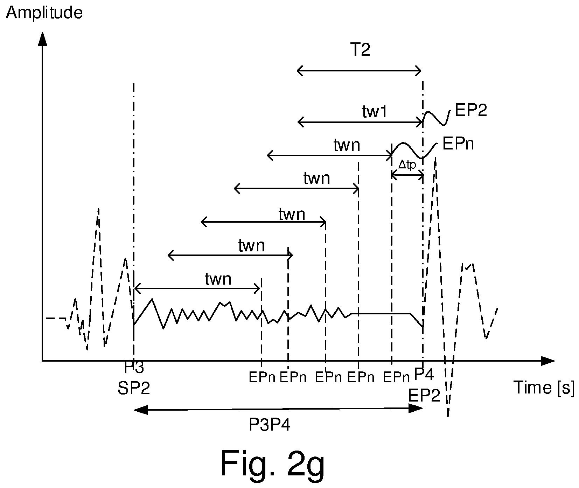

Fig. 2g discloses a diagram comprising a number of time windows distributed over a cardiac cycle X according to one aspect of the invention.The method according to claim 8 or 9 used to analyze murmur in a cardiac cycle X is described in relation to said diagram. Said method is used to further analyze said confirmed pathological murmur in a cardiac cycle X.

Said phonocardiogram comprises one cardiac cycle X where pathological murmur is displayed. As a first step, a third point P3 in relation to a first heart sound S1 in said cardiac cycle X is determined. Thereafter, a fourth point P4 in relation to a second heart sound S2 in said cardiac cycle X is determined. A first analysis on said heart sound over a time window tw1 is performed, said time window tw1 having an ending point EP2 essentially the same as said fourth point P4 and having a duration of a second predetermined time T2. At least one further analysis on said heart sounds over a second time window twn is performed, said time window twn having an ending point EPn essentially a predetermined time period Δtp earlier than said ending point EP2, EPn of said previous time window tw1, twn and having a duration of said second predetermined time T2.

Additional time windows twn are created and analyses on the heart sound over said additional time windows twn are performed, until said time window twn has a starting point SP2 essentially the same as said third point P3.

The analysis method described above can be performed on each cardiac cycle X of a phonocardiogram.

Based on the results from the analysis above on said time windows tw1, twn a confirmation or a dismissing of said predetermined pathological condition of said heart sound is made.

Fig. 3a schematically illustrates a flow diagram of a method performed in a processing unit 220 of the device 200 for classifying heart sounds presented in a phonocardiogram.

In a first method step S100 a phonocardiogram is received. Said phonocardiogram comprises at least one cardiac cycle X. Said phonocardiogram could according to one embodiment be received in said processor 200 via a wireless communication. Said phonocardiogram comprises at least one cardiac cycle X which is selected for further analysis.

In method step S110, the two major heart sounds of said at least one cardiac cycle X are determined, S1 when said first set of valves of said heart closes, and S2 when said second set of valves of said heart closes. A first point P1 in relation to S1 is determined.

In method step S120, a second point P2 in relation to S2 is determined.

In method step S130, a first analysis on said heart sound over a first time window w1 is performed, said first time window w1 having a starting point SP1 of essentially said first point P1 or having an ending point EP1 of essentially said second point P2 and having a duration of a first predetermined time T1, wherein said first predetermined time T1 is shorter than a time period P1P2 between said first point P1 and said second point P2. Said analysis could according to one embodiment comprise the step of extracting spectral energies from said phonocardiogram.

In method step S140, one further analysis on said heart sound over one further time window wn is performed, said further time window wn having a starting point SP1 of essentially said first point P1 or having an ending point EP1 of essentially said second point P2 and having a duration of a predetermined time Tn. Said predetermined time Tn is the sum of the duration of said previous time window w1, wn and a predetermined time period Δt. Said analysis could according to one embodiment comprise the step of extracting spectral energies from said phonocardiogram.

In method step S150 the duration of the next time window, Tn+ Δt, is compared with the interval determined by the first point P1 and the second point P2, P1P2. When the duration of said next time window Tn is shorter than P1P2, step 140 is repeated.

When the duration of said next time window Tn is equal or larger than P1P2, a next step S160 is performed.

Method step S160 involves a classification of said heart sound as a first or a second heart condition. Said first heart condition is according to one embodiment a non-pathological condition. Said second heart condition is according to one embodiment a pathological condition. Said classification is made based on the results from said previous analysis, that is, said extracted spectral features. According to one embodiment, said classification involves using a support vector machine method.

Said method steps can according to one embodiment be performed in a different order than the one described above.

Fig. 3a schematically illustrates a flow diagram of a method performed in a processing unit 220 of the device 200 for classifying heart sounds presented in a phonocardiogram.

When a pathological condition has been determined, a second analysis can be performed on said phonocardiogram in order to determine which specific pathological condition the heart sounds on said phonocardiogram implies, e.g. if the pathological condition is aortic stenosis, aortic regurgitation, mitral insufficiency or any other type of pathological condition that may be determined from an analysis of phonocardiograms.

In a first method step S200 a predetermined pathological condition is assumed.

In method step S210, said phonocardiogram is received. Said phonocardiogram comprises at least one cardiac cycle X which is selected for further analysis. Said phonocardiogram could according to one embodiment be sent to said processor 200 via a wireless communication. In method step S210, the two major heart sounds of said at least one cardiac cycle X are determined, S1 when said first set of valves of said heart closes, and S2 when said second set of valves of said heart closes. A third point P3 in relation to S1 is determined.

In method step S230, a fourth point P4 in relation to S2 is determined.

In method step S240, a first analysis on said heart sound over a first time window tw1 is performed, said first time window tw1 having a starting point SP2 of essentially said third point P3 or having an ending point EP2 of essentially said fourth point P4 and having a duration of a second predetermined time T2, wherein said second predetermined time T2 is shorter than a time period P3P4 between said third point P3 and said fourth point P4. Said analysis could according to one embodiment comprise the step of extracting spectral energies from said phonocardiogram.

In method step S250, one further analysis on said heart sound over at least one further first time window twn is performed, said further time window twn having a starting point SPn a predetermined time period Δtp later than said starting point SP2, SPn of said previous time window tw1, twn or having an ending point EPn a predetermined time period Δtp earlier than said ending point EP2, EPn of said previous time window tw1, twn. Said analysis could according to one embodiment comprise the step of extracting spectral energies from said phonocardiogram. Said first analysis and at least one further analysis according comprises, according to one embodiment, extracting spectral energies from said heart sound.

In method step S260 the placement over said phonocardiogram of the next time window is controlled. When the next time window twn is placed within the interval determined by the third point P3 and the fourth point P4, P3P4, where said placement of said time window twn is performed according to the method as described above, step S250 is repeated.

When the next time window twn is placed on the starting point P3 or the ending point P4 of said interval P3P4 or when a part of said time window twn is placed outside said interval P3P4, a subsequent step 270 is performed.

In method step S270 said assumed predetermined pathological condition is confirmed or dismissed on the basis of said first analysis and said at least one further analysis. Said confirmation or dismissal of said predetermined pathological condition of said heart sound involves according to one embodiment using a support vector machine method.

Said method steps can according to one embodiment be performed in a different order than the one described.

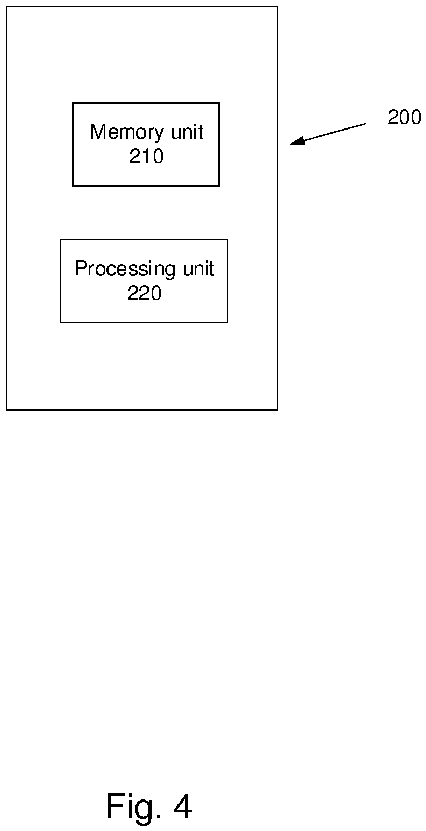

Fig. 4 schematically illustrates the device 200 for classifying heart sounds. Said device 200 comprises a memory unit 210 and a processing unit 220. In said memory unit 210, a computer program P is stored in order to control the function of said device 200.

Said computer program P, comprising computer program code, is arranged to receiving S100 a phonocardiogram comprising at least one cardiac cycle X, determining S110 a first point P1 in relation to a first heart sound S1 in said cardiac cycle X, determining S120 a second point P2 in relation to a second heart sound S2 in said cardiac cycle X, performing S130 a first analysis on said heart sound over a first time window w1, said first time window w1 having a starting point SP1 essentially the same as said first point P1 or an endpoint EP1 essentially the same as said second point P2 and having a duration of a first predetermined time T1, wherein said first predetermined time T1 is shorter than a time period P1P2 between said first point P1 and said second point P2. When at least one further time window wn has a duration of a further predetermined time Tn and said further predetermined time Tn is shorter than said time period P1P2, at least one further analysis on said heart sound over at least one further time window wn is performed, said at least one further time window wn having essentially the same starting point SP1 as said first point P1 or essentially the same ending point EP1 as said second point P2 and having a duration of a further predetermined time Tn, wherein the duration of said second time window wn is the duration T1, Tn of said previous time window w1, wn plus a predetermined time period Δt. Further, a classification of said heart sound as a first or a second heart condition, on the basis of said first and the at least one further analysis of the heart sound over said first and at least one further time windows w1, wn, is performed.

While the invention has been particularly shown and described with reference to preferred embodiments thereof, it will be understood by those skilled in the relevant art that various modifications with regard to form and detail may be performed without departing from the scope of the invention, which is defined in the appended claims.

## Claims

1. A device (200) for classifying heart sounds presented in a phonocardiogram comprising:
a memory unit (210) and a processing unit (220) configured to classify heart sounds presented in a phonocardiogram by performing the steps of:
- receiving (S100) a phonocardiogram comprising at least one cardiac cycle (X);
- determining (S110) a first point (P1) in relation to a first heart sound (S1) in said cardiac cycle (X), presented in the phonocardiogram;
- determining (S120) a second point (P2) in relation to a second heart sound (S2) in said cardiac cycle (X), presented in the phonocardiogram;
- performing (S130) a first analysis on said heart sounds over a first time window (w1), said analysis comprising the step of extracting spectral energies from said phonocardiogram, said first time window (w1) having a starting point (SP1) essentially the same as said first point (P1) or an endpoint (EP1) essentially the same as said second point (P2) and having a duration of a first predetermined time (T1), wherein said first predetermined time (T1) is shorter than a time period (P1P2) between said first point (P1) and said second point (P2);
- when at least one further time window (wn) has a duration of a further predetermined time (Tn) wherein said further predetermined time (Tn) is shorter than said time period (P1P2), performing at least one further analysis (S140) on said heart sound over at least one further time window (wn), said analysis comprising the step of extracting spectral energies from said phonocardiogram, said at least one further time window (wn) having essentially the same starting point (SP1) as said first point (P1) or essentially the same ending point (EP1) as said second point (P2), wherein said predetermined time (Tn) is the sum of the duration of a previous time window (w1, wn) and a predetermined time period (Δt); and
- classifying said heart sound (S160), presented in the phonocardiogram, as a non-pathological or a pathological heart condition on the basis of said first and the at least one further analysis of the heart sound over said first and at least one further time windows (w1,wn);
**characterized in that** the first predetermined time (T1) and the predetermined time period are determined based on data from a database comprising phonocardiograms from several hearts wherein the phonocardiograms have been confirmed to belong to one out of three groups: no murmur, innocent murmur and pathological murmur.

2. The device according to claim 1 wherein said classification of the heart sound into a first or second heart condition involves using a support vector machine method.

3. The device according any of the previous claims, wherein said phonocardiogram comprises several cardiac cycles (X), and the processing unit (200) is configured to:
- derive a result from said analysis of the heart sound from said first and at least one further time windows (w1, wn) for each cardiac cycle (X);
- derive an average result on the basis of the result from each of the analysis of the cardiac cycles (X)
- classify the heart sound into a first or second heart condition on the basis of said derived average result.

4. The device according to any of the previous claims wherein said phonocardiogram comprises several cardiac cycles (X) wherein the classification of the heart sound into a first or second heart condition for each cardiac cycle (X) is carried out for each cardiac cycle (X) and used in order to determine an overall classification for the phonocardiogram.

5. The device according to any of the previous claims, wherein the processing unit (200) is configured to perform a further analysis of the heart sound, upon the heart sound presented in the phonocardiogram being classified as a pathological heart condition, said further analysis comprising the further steps of:
- assuming that said pathological condition is a predetermined pathological condition;
- receiving said phonocardiogram comprising at least one cardiac cycle (X);
- determining a third point (P3) in relation to a first heart sound (S1) in said cardiac cycle (X);
- determining a fourth point (P4) in relation to a second heart sound (S2) in said cardiac cycle (X);
- performing a first analysis on said heart sound over a time window (tw1), said time window (tw1) having a starting point (SP2) essentially the same as said third point (P3) or having an ending point (EP2) essentially the same as said fourth point (P4) and having a duration of a second predetermined time (T2);
- when one at least additional time window (twn), said time window (twn) having a duration of said second predetermined time (T2), is within a time interval created between said third (P3) and fourth (P4) points, performing at least one further analysis on said heart sound over said at least one additional time window (twn), said at least one additional time window (twn) having a starting point (SPn) a time period (Δtp) later than said starting point (SP2, SPn) of said previous time window (tw, twn) or having an ending point (EPn) a predetermined time period (Δtp) earlier than said ending point (EP2, EPn) of said previous time window (tw1, twn);
- confirming or dismissing said predetermined pathological condition of said heart sound on the basis of said first and at least one further analysis of the heart sound over said first and at least one additional time window (tw1, twn).

6. The device according to claim 5, wherein the processors is configured to perform the further steps of:
- grading the severity of said predetermined pathological condition of said heart sound on the basis of said first and at least one further analysis of the heart sound over said first and at least one additional time window (tw1, twn).

7. The device according to claim 6 wherein said confirmation or dismissal of said predetermined pathological condition of said heart sound involves using a support vector machine method.

8. The device according to claim 7 wherein said grading of the severity of said predetermined pathological condition of said heart sound involves using a wavelet analysis or stepwise regression analysis.

9. The device according to any of claims 5-8 wherein said pathological condition is aortic stenosis.

10. The device according to any of claims 5-8 wherein said pathological condition is aortic regurgitation.

11. The device according to any of claims 5-8 wherein said pathological condition is mitral insufficiency.

12. The device according to any of the previous claims wherein said first or third points (P1, P3) and said second or fourth points (P2, P4) occur within the systolic duration of a cardiac cycle (X).

13. A computer program (P) comprising computer program code which, when executed in a processing unit of a device for classifying heart sounds according to claim 1, causes the processing unit (220) to execute the following steps:
- receive (S100) a phonocardiogram comprising at least one cardiac cycle (X);
- determine (S110) a first point (P1) in relation to a first heart sound (S1) in said cardiac cycle (X), presented in the phonocardiogram;
- determine (S120) a second point (P2) in relation to a second heart sound (S2) in said cardiac cycle (X), presented in the phonocardiogram;
- perform (S130) a first analysis on said heart sounds over a first time window (w1), said analysis comprising the step of extracting spectral energies from said phonocardiogram, said first time window (w1) having a starting point (SP1) essentially the same as said first point (P1) or an endpoint (EP1) essentially the same as said second point (P2) and having a duration of a first predetermined time (T1), wherein said first predetermined time (T1) is shorter than a time period (P1P2) between said first point (P1) and said second point (P2);
- when at least one further time window (wn) has a duration of a further predetermined time (Tn) wherein said further predetermined time (Tn) is shorter than said time period (P1P2), perform at least one further analysis (S140) on said heart sound over at least one further time window (wn), said analysis comprising the step of extracting spectral energies from said phonocardiogram, said at least one further time window (wn) having essentially the same starting point (SP1) as said first point (P1) or essentially the same ending point (EP1) as said second point (P2), wherein said predetermined time (Tn) is the sum of the duration of a previous time window (w1, wn) and a predetermined time period (Δt);
- classify said heart sound (S160), presented in the phonocardiogram, as a non-pathological or a pathological heart condition on the basis of said first and the at least one further analysis of the heart sound over said first and at least one further time windows (w1,wn);
**characterized in that** said first predetermined time (T1) and said predetermined time period are determined based on data from a database comprising phonocardiograms from several hearts wherein said phonocardiograms have been confirmed to belong to one out of three groups: no murmur, innocent murmur and pathological murmur.

## Patentansprüche

1. Vorrichtung (200) zum Klassifizieren von Herztönen, die in einem Phonokardiogramm dargestellt werden, die Folgendes umfasst:
eine Speichereinheit (210) und eine Verarbeitungseinheit (220), die dafür konfiguriert ist, Herztöne, die in einem Phonokardiogramm dargestellt werden, durch Durchführen der folgenden Schritte zu klassifizieren:
- Empfangen (S100) eines Phonokardiogramms, das wenigstens einen Herzzyklus (X) umfasst;
- Bestimmen (S110) eines ersten Punkts (P1) in Beziehung zu einem ersten Herzton (S1) in dem Herzzyklus (X), der in dem Phonokardiogramm dargestellt wird;
- Bestimmen (S120) eines zweiten Punkts (P2) in Beziehung zu einem zweiten Herzton (S2) in dem Herzzyklus (X), der in dem Phonokardiogramm dargestellt wird;
- Durchführen (S130) einer ersten Analyse an den Herztönen über ein erstes Zeitfenster (w1), wobei die Analyse den Schritt des Extrahierens von Spektralenergien aus dem Phonokardiogramm umfasst, wobei das erste Zeitfenster (w1) einen Startpunkt (SP1), der im Wesentlichen der gleiche ist wie der erste Punkt (P1), oder einen Endpunkt (EP1), der im Wesentlichen der gleiche ist wie der zweite Punkt (P2), aufweist und eine Dauer einer ersten vorbestimmten Zeit (T1) aufweist, wobei die erste vorbestimmte Zeit (T1) kürzer ist als ein Zeitraum (P1P2) zwischen dem ersten Punkt (P1) und dem zweiten Punkt (P2);
- wenn wenigstens ein weiteres Zeitfenster (wn) eine Dauer einer weiteren vorbestimmten Zeit (Tn) aufweist, wobei die weitere vorbestimmte Zeit (Tn) kürzer ist als der Zeitraum (P1P2), Durchführen wenigstens einer weiteren Analyse (S140) an dem Herzton über wenigstens ein weiteres Zeitfenster (wn), wobei die Analyse den Schritt des Extrahierens von Spektralenergien aus dem Phonokardiogramm umfasst, wobei das wenigstens eine weitere Zeitfenster (wn) den Startpunkt (SP1), der im Wesentlichen der gleiche ist wie der erste Punkt (P1), oder den Endpunkt (EP1), der im Wesentlichen der gleiche ist wie der zweite Punkt (P2), aufweist, wobei die vorbestimmte Zeit (Tn) die Summe der Dauer eines vorhergehenden Zeitfensters (w1, wn) und eines vorbestimmten Zeitraums (Δt) ist; und
- Klassifizieren des Herztons (160), der in dem Phonokardiogramm dargestellt wird, als einen nicht-pathologischen oder einen pathologischen Herzzustand auf der Grundlage der ersten und der wenigstens einen weiteren Analyse des Herztons über das erste und wenigstens ein weiteres Zeitfenster (w1, wn);
**dadurch gekennzeichnet, dass** die erste vorbestimmte Zeit (T1) und der vorbestimmte Zeitraum auf Grundlage von Daten von einer Datenbank bestimmt werden, die Phonokardiogramme von mehreren Herzen umfasst, wobei bestätigt worden ist, dass die Phonokardiogramme zu einer von drei Gruppen gehören: kein Geräusch, harmloses Geräusch und pathologisches Geräusch.

2. Vorrichtung nach Anspruch 1, wobei die Klassifizierung des Herztons zu einem ersten oder einem zweiten Herzzustand die Verwendung eines Support-Vektor-Maschinen-Verfahrens einschließt.

3. Vorrichtung nach einem der vorhergehenden Ansprüche, wobei das Phonokardiogramm mehrere Herzzyklen (X) umfasst und die Verarbeitungseinheit (220) zu Folgendem konfiguriert ist:
- aus der Analyse des Herztons aus dem ersten und wenigstens einem weitere Zeitfenster (w1, wn) für jeden Herzzyklus (X) ein Ergebnis abzuleiten;
- auf der Grundlage des Ergebnisses aus jeder der Analysen der Herzzyklen (X) ein durchschnittliches Ergebnis abzuleiten;
- den Herzton auf Grundlage des abgeleiteten durchschnittlichen Ergebnisses zu einem ersten oder einem zweiten Herzzustand zu klassifizieren.

4. Vorrichtung nach einem der vorhergehenden Ansprüche, wobei das Phonokardiogramm mehrere Herzzyklen (X) umfasst, wobei die Klassifizierung des Herztons zu einem ersten oder einem zweiten Herzzustand für jeden Herzzyklus (X) für jeden Herzzyklus (X) ausgeführt und verwendet wird, um eine Gesamtklassifikation für das Phonokardiogramm zu bestimmen.

5. Vorrichtung nach einem der vorhergehenden Ansprüche, wobei die Verarbeitungseinheit (220) dafür konfiguriert ist, daraufhin, dass der Herzton, der in dem Phonokardiogramm dargestellt wird, als ein pathologischer Herzzustand klassifiziert wird, eine weitere Analyse des Herztons durchzuführen, wobei die weitere Analyse die folgenden weiteren Schritte umfasst:
- Annehmen, dass der pathologische Zustand ein vorbestimmter pathologischer Zustand ist;
- Empfangen des Phonokardiogramms, das wenigstens einen Herzzyklus (X) umfasst;
- Bestimmen eines dritten Punkts (P3) in Beziehung zu einem ersten Herzton (S1) in dem Herzzyklus (X);
- Bestimmen eines vierten Punkts (P4) in Beziehung zu einem zweiten Herzton (S2) in dem Herzzyklus (X) ;
- Durchführen einer ersten Analyse an den Herztönen über ein erstes Zeitfenster (tw1), wobei das Zeitfenster (tw1) einen Startpunkt (SP2), der im Wesentlichen der gleiche ist wie der dritte Punkt (P3), oder einen Endpunkt (EP2), der im Wesentlichen der gleiche ist wie der vierte Punkt (P4), aufweist und eine Dauer einer zweiten vorbestimmten Zeit (T2) aufweist;
- wenn wenigstens ein zusätzliches Zeitfenster (twn), wobei das Zeitfenster (twn) eine Dauer einer zweiten vorbestimmten Zeit (T2) aufweist, innerhalb eines Zeitraums liegt, der zwischen dem dritten (P3) und dem vierten (P4) Punkt erzeugt wird, Durchführen wenigstens einer weiteren Analyse an dem Herzton über das wenigstens eine zusätzliche Zeitfenster (twn), wobei das wenigstens eine zusätzliche Zeitfenster (twn) einen Startpunkt (SPn), einen Zeitraum (Δtp) später als der Startpunkt (SP2, SPn) des vorhergehenden Zeitfensters (tw, twn), oder einen Endpunkt (EPn), einen Zeitraum (Δtp) früher als der Endpunkt (EP2, EPn) des vorhergehenden Zeitfensters (tw1, twn), aufweist;
- Bestätigen oder Ablehnen des vorbestimmten pathologischen Zustandes des Herztons auf der Grundlage der ersten und der wenigstens einen weiteren Analyse des Herztons über das erste und das wenigstens eine zusätzliche Zeitfenster (tw1, twn) .

6. Vorrichtung nach Anspruch 5, wobei der Prozessor dafür konfiguriert ist, die folgenden weiteren Schritte durchzuführen:
- Einstufen der Schwere des vorbestimmten pathologischen Zustandes des Herztons auf der Grundlage der ersten und der wenigstens einen weiteren Analyse des Herztons über das erste und das wenigstens eine zusätzliche Zeitfenster (tw1, twn) .

7. Vorrichtung nach Anspruch 6, wobei die Bestätigung oder Ablehnung des vorbestimmten pathologischen Zustandes des Herztons die Verwendung eines Support-Vektor-Maschinen-Verfahrens einschließt.

8. Vorrichtung nach Anspruch 7, wobei das Einstufen der Schwere des vorbestimmten pathologischen Zustandes des Herztons die Verwendung einer Wavelet-Analyse oder einer schrittweisen Regressionsanalyse einschließt.

9. Vorrichtung nach einem der Ansprüche 5 bis 8, wobei der pathologische Zustand eine Aortenstenose ist.

10. Vorrichtung nach einem der Ansprüche 5 bis 8, wobei der pathologische Zustand eine Aortenregurgitation ist.

11. Vorrichtung nach einem der Ansprüche 5 bis 8, wobei der pathologische Zustand eine Mitralinsuffizienz ist.

12. Vorrichtung nach einem der vorhergehenden Ansprüche, wobei der erste oder der dritte Punkt (P1, P3) und der zweite oder der vierte Punkt (P2, P4) innerhalb der systolischen Dauer eines Herzzyklus (X) auftreten.

13. Rechnerprogramm (P), das Rechnerprogrammcode umfasst, der, wenn er in einer Verarbeitungseinheit einer Vorrichtung zum Klassifizieren von Herztönen nach Anspruch 1 ausgeführt wird, veranlasst, dass die Verarbeitungseinheit (220) die folgenden Schritte ausführt:
- Empfangen (S100) eines Phonokardiogramms, das wenigstens einen Herzzyklus (X) umfasst;
- Bestimmen (S110) eines ersten Punkts (P1) in Beziehung zu einem ersten Herzton (S1) in dem Herzzyklus (X), der in dem Phonokardiogramm dargestellt wird;
- Bestimmen (S120) eines zweiten Punkts (P2) in Beziehung zu einem zweiten Herzton (S2) in dem Herzzyklus (X), der in dem Phonokardiogramm dargestellt wird;
- Durchführen (S130) einer ersten Analyse an den Herztönen über ein erstes Zeitfenster (w1), wobei die Analyse den Schritt des Extrahierens von Spektralenergien aus dem Phonokardiogramm umfasst, wobei das erste Zeitfenster (w1) einen Startpunkt (SP1), der im Wesentlichen der gleiche ist wie der erste Punkt (P1), oder einen Endpunkt (EP1), der im Wesentlichen der gleiche ist wie der zweite Punkt (P2), aufweist und eine Dauer einer ersten vorbestimmten Zeit (T1) aufweist, wobei die erste vorbestimmte Zeit (T1) kürzer ist als ein Zeitraum (P1P2) zwischen dem ersten Punkt (P1) und dem zweiten Punkt (P2);
- wenn wenigstens ein weiteres Zeitfenster (wn) eine Dauer einer weiteren vorbestimmten Zeit (Tn) aufweist, wobei die weitere vorbestimmte Zeit (Tn) kürzer ist als der Zeitraum (P1P2), Durchführen wenigstens einer weiteren Analyse (S140) an dem Herzton über wenigstens ein weiteres Zeitfenster (wn), wobei die Analyse den Schritt des Extrahierens von Spektralenergien aus dem Phonokardiogramm umfasst, wobei das wenigstens eine weitere Zeitfenster (wn) den Startpunkt (SP1), der im Wesentlichen der gleiche ist wie der erste Punkt (P1), oder den Endpunkt (EP1), der im Wesentlichen der gleiche ist wie der zweite Punkt (P2), aufweist, wobei die vorbestimmte Zeit (Tn) die Summe der Dauer eines vorhergehenden Zeitfensters (w1, wn) und eines vorbestimmten Zeitraums (Δt) ist;
- Klassifizieren des Herztons (S160), der in dem Phonokardiogramm dargestellt wird, als einen nicht-pathologischen oder einen pathologischen Herzzustand auf der Grundlage der ersten und der wenigstens einen weiteren Analyse des Herztons über das erste und wenigstens ein weiteres Zeitfenster (w1, wn);
**dadurch gekennzeichnet, dass** die erste vorbestimmte Zeit (T1) und der vorbestimmte Zeitraum auf Grundlage von Daten von einer Datenbank bestimmt werden, die Phonokardiogramme von mehreren Herzen umfasst, wobei bestätigt worden ist, dass die Phonokardiogramme zu einer von drei Gruppen gehören: kein Geräusch, harmloses Geräusch und pathologisches Geräusch.

## Revendications

1. Dispositif (200) pour la classification des bruits du cœur présenté dans un phonocardiogramme comprenant :
une unité de mémoire (210) et une unité de traitement (220) configurée pour classifier les bruits du cœur présentés dans un phonocardiogramme en exécutant les étapes de :
- recevoir (S100) un phonocardiogramme comprenant au moins un cycle cardiaque (X) ;
- déterminer (S110) un premier point (P1) par rapport à un premier bruit du cœur (S1) dans ledit cycle cardiaque (X), présenté dans le phonocardiogramme ;
- déterminer (S120) un deuxième point (P2) par rapport à un second bruit du cœur (S2) dans ledit cycle cardiaque (X), présenté dans le phonocardiogramme ;
- exécuter (S130) une première analyse sur lesdits bruits du cœur sur une première fenêtre temporelle (w1), ladite analyse comprenant l'étape d'extraire des énergies spectrales dudit phonocardiogramme, ladite première fenêtre temporelle (w1) ayant un point de départ (SP1) essentiellement le même que ledit premier point (P1) ou un point de fin (EP1) essentiellement le même que ledit deuxième point (P2) et ayant une durée d'un premier temps prédéterminé (T1), dans lequel ledit premier temps prédéterminé (T1) est plus court qu'une période de temps (P1P2) entre ledit premier point (P1) et ledit deuxième point (P2) ;
- lorsqu'au moins une fenêtre temporelle supplémentaire (wn) a une durée d'un temps prédéterminé supplémentaire (Tn) dans lequel ledit temps prédéterminé supplémentaire (Tn) est plus court que ladite période de temps (P1P2), exécuter au moins une analyse supplémentaire (S140) sur ledit bruit du cœur sur au moins une fenêtre temporelle supplémentaire (wn), ladite analyse comprenant l'étape d'extraire des énergies spectrales dudit phonocardiogramme, ladite au moins une fenêtre temporelle supplémentaire (wn) ayant essentiellement le même point de départ (SP1) que ledit premier point (P1) ou essentiellement le même point de fin (EP1) que ledit deuxième point (P2), dans lequel ledit temps prédéterminé (Tn) est la somme de la durée d'une fenêtre temporelle (w1, wn) précédente et d'une période de temps prédéterminée (Δt) ; et
- classifier ledit bruit du cœur (S160), présenté dans le phonocardiogramme, en tant qu'un trouble cardiaque non-pathologique ou pathologique en se basant sur ladite première et ladite au moins une analyse supplémentaire du bruit du cœur sur ladite première et au moins une fenêtre temporelle (w1, wn) supplémentaire ;
**caractérisé en ce que** le premier temps prédéterminé (T1) et la période de temps prédéterminée sont déterminés en se basant sur des données provenant d'une base de données comprenant des phonocardiogrammes issus de plusieurs cœurs, dans lequel les phonocardiogrammes ont été confirmés comme appartenant à un sur trois groupes : pas de murmure, murmure innocent et murmure pathologique.

2. Dispositif selon la revendication 1, dans lequel ladite classification du bruit du cœur en un premier ou second trouble cardiaque implique d'utiliser un procédé par machine à vecteurs de support.

3. Dispositif selon l'une quelconque des revendications précédentes, dans lequel ledit phonocardiogramme comprend plusieurs cycles cardiaques (X), et l'unité de traitement (220) est configurée pour :
- dériver un résultat de ladite analyse du bruit du cœur à partir de ladite première et d'au moins une fenêtre temporelle (w1, wn) supplémentaire pour chaque cycle cardiaque (X) ;
- dériver un résultat moyen en se basant sur le résultat de chaque analyse des cycles cardiaques (X) ;
- classifier le bruit du cœur en un premier ou second trouble cardiaque en se basant sur ledit résultat moyen dérivé.

4. Dispositif selon l'une quelconque des revendications précédentes, dans lequel ledit phonocardiogramme comprend plusieurs cycles cardiaques (X), dans lequel la classification du bruit du cœur en un premier ou second trouble cardiaque pour chaque cycle cardiaque (X) est exécutée pour chaque cycle cardiaque (X) et utilisée afin de déterminer une classification globale pour le phonocardiogramme.

5. Dispositif selon l'une quelconque des revendications précédentes, dans lequel l'unité de traitement (220) est configurée pour exécuter une analyse supplémentaire du bruit du cœur, au moment où le bruit du cœur présenté dans le phonocardiogramme est classifié en tant qu'un trouble cardiaque pathologique, ladite analyse supplémentaire comprenant les étapes supplémentaires de :
- supposer que ledit trouble pathologique est un trouble pathologique prédéterminé ;
- recevoir ledit phonocardiogramme comprenant au moins un cycle cardiaque (X) ;
- déterminer un troisième point (P3) par rapport à un premier bruit du cœur (S1) dans ledit cycle cardiaque (X) ;
- déterminer un quatrième point (P4) par rapport à un second bruit du cœur (S2) dans ledit cycle cardiaque (X) ;
- exécuter une première analyse sur ledit bruit du cœur sur une fenêtre temporelle (tw1), ladite fenêtre temporelle (tw1) ayant un point de départ (SP2) essentiellement le même que ledit troisième point (P3) ou ayant un point de fin (EP2) essentiellement le même que ledit quatrième point (P4) et ayant une durée d'un second temps prédéterminé (T2) ;
- lorsqu'au moins une fenêtre temporelle supplémentaire (twn), ladite fenêtre temporelle (twn) ayant une durée dudit second temps prédéterminé (T2), est comprise dans un intervalle de temps créé entre lesdits troisième (P3) et quatrième (P4) points, exécuter au moins une analyse supplémentaire sur ledit bruit du cœur sur ladite au moins une fenêtre temporelle supplémentaire (twn), ladite au moins une fenêtre temporelle supplémentaire (twn) ayant un point de départ (SPn) une période de temps (Δtp) plus tard que ledit point de départ (SP2, SPn) de ladite fenêtre temporelle (tw, twn) précédente ou ayant un point de fin (EPn) une période de temps prédéterminée (Δtp) plus tôt que ledit point de fin (EP2, EPn) de ladite fenêtre temporelle (tw1, twn) précédente ;
- confirmer ou exclure ledit trouble pathologique prédéterminé dudit bruit du cœur en se basant sur ladite première et au moins une analyse supplémentaire du bruit du cœur sur ladite première et au moins une fenêtre temporelle (tw1, twn) supplémentaire.

6. Dispositif selon la revendication 5, dans lequel le processeur est configuré pour exécuter les étapes supplémentaires de :
- classer la gravité dudit trouble pathologique prédéterminé dudit bruit du cœur en se basant sur ladite première et au moins une analyse supplémentaire du bruit du cœur sur ladite première et au moins une fenêtre temporelle (tw1, twn) supplémentaire.

7. Dispositif selon la revendication 6, dans lequel ladite confirmation ou ladite exclusion dudit trouble pathologique prédéterminé dudit bruit du cœur implique d'utiliser un procédé par machine à vecteurs de support.

8. Dispositif selon la revendication 7, dans lequel ledit classement de la gravité dudit trouble pathologique prédéterminé dudit bruit du cœur implique d'utiliser une analyse par ondelettes ou une analyse par régression pas à pas.

9. Dispositif selon l'une quelconque des revendications 5 - 8, dans lequel ledit trouble pathologique est une sténose aortique.

10. Dispositif selon l'une quelconque des revendications 5 - 8, dans lequel ledit trouble pathologique est une régurgitation aortique.

11. Dispositif selon l'une quelconque des revendications 5 - 8, dans lequel ledit trouble pathologique est une insuffisance mitrale.

12. Dispositif selon l'une quelconque des revendications précédentes, dans lequel lesdits premier ou troisième points (P1, P3) et lesdits deuxième ou quatrième points (P2, P4) se produisent pendant la durée systolique d'un cycle cardiaque (X).

13. Programme informatique (P) comprenant un code de programme informatique qui, lorsqu'il est exécuté dans une unité de traitement d'un dispositif pour la classification des bruits du cœur selon la revendication 1, fait que l'unité de traitement (220) exécute les étapes suivantes :
- recevoir (S100) un phonocardiogramme comprenant au moins un cycle cardiaque (X) ;
- déterminer (S110) un premier point (P1) par rapport à un premier bruit du cœur (S1) dans ledit cycle cardiaque (X), présenté dans le phonocardiogramme ;
- déterminer (S120) un deuxième point (P2) par rapport à un second bruit du cœur (S2) dans ledit cycle cardiaque (X), présenté dans le phonocardiogramme ;
- exécuter (S130) une première analyse sur lesdits bruits du cœur sur une première fenêtre temporelle (w1), ladite analyse comprenant l'étape d'extraire des énergies spectrales dudit phonocardiogramme, ladite première fenêtre temporelle (w1) ayant un point de départ (SP1) essentiellement le même que ledit premier point (P1) ou un point de fin (EP1) essentiellement le même que ledit deuxième point (P2) et ayant une durée d'un premier temps prédéterminé (T1), dans lequel ledit premier temps prédéterminé (T1) est plus court qu'une période de temps (P1P2) entre ledit premier point (P1) et ledit deuxième point (P2) ;
- lorsqu'au moins une fenêtre temporelle supplémentaire (wn) a une durée d'un temps prédéterminé supplémentaire (Tn) dans lequel ledit temps prédéterminé supplémentaire (Tn) est plus court que ladite période de temps (P1P2), exécuter au moins une analyse supplémentaire (S140) sur ledit bruit du cœur sur au moins une fenêtre temporelle supplémentaire (wn), ladite analyse comprenant l'étape d'extraire des énergies spectrales dudit phonocardiogramme, ladite au moins une fenêtre temporelle supplémentaire (wn) ayant essentiellement le même point de départ (SP1) que ledit premier point (P1) ou essentiellement le même point de fin (EP1) que ledit deuxième point (P2), dans lequel ledit temps prédéterminé (Tn) est la somme de la durée d'une fenêtre temporelle (w1, wn) précédente et d'une période de temps prédéterminée (Δt) ; et
- classifier ledit bruit du cœur (S160), présenté dans le phonocardiogramme, en tant qu'un trouble cardiaque non-pathologique ou pathologique en se basant sur ladite première et ladite au moins une analyse supplémentaire du bruit du cœur sur ladite première et au moins une fenêtre temporelle (w1, wn) supplémentaire ;
**caractérisé en ce que** le premier temps prédéterminé (T1) et ladite période de temps prédéterminée sont déterminés en se basant sur des données provenant d'une base de données comprenant des phonocardiogrammes issus de plusieurs cœurs, dans lequel lesdits phonocardiogrammes ont été confirmés comme appartenant à un sur trois groupes : pas de murmure, murmure innocent et murmure pathologique.
